# EUROPÄISCHE PATENTSCHRIFT

(11) **EP 0 972 533 B1**
(45) Veröffentlichungstag und Bekanntmachung des Hinweises auf die Patenterteilung: **23.03.2005**
(21) Anmeldenummer: 99113227.5
(22) Anmeldetag: 08.07.1999
(51) Int. Cl.: A61M 15/00, A61M 15/08, B05B 11/00, G01F 11/02

(54) **Spender für Medien**
Product dispenser
Distributeur de produit

(30) Priorität: 14.07.1998 DE 19831525
(43) Veröffentlichungstag der Anmeldung: 19.01.2000
(73) Patentinhaber: ING. ERICH PFEIFFER GMBH, 78315 Radolfzell (DE)
(72) Erfinder: Ritsche, Stefan, 78315 Radolfzell (DE); Käfer, Stefan, 78253 Eigeltingen (DE)
(74) Vertreter: Patentanwälte Ruff, Wilhelm, Beier, Dauster & Partner

(56) Entgegenhaltungen:
- WO-A-98/03217
- US-A- 4 627 432
- US-A- 5 388 572
- US-A- 5 562 918
- US-A- 5 584 417
- US-A- 5 622 166

## Beschreibung

Die Erfindung betrifft einen Spender, insbesondere zum Austrag fließfähiger Medien. Diese können gasförmig, pastös oder flüssig, vorzugsweise pulverförmig sein. Beim Medienaustrag ist der Spender mit einer einzigen. Hand zu halten und gleichzeitig zu betätigen. Das Medium kann durch Saugen bzw. Einatmen gefördert werden. Dabei kann der enthaltene pharmazeutische Wirkstoff inhaliert oder auf der Nasenschleimhaut abgelagert werden. Der Spender kann vollständig aus Spritzguß- bzw. Kunststoffteilen bestehen.

Die US 5,584,417 A beschreibt einen Spender nach dem Oberbegriff des Anspruchs 1. Sein einer Grundkörper trägt eine Nadel, mit der durch eine Membran verschlossene Stopfen von Ampullen durchstossen werden können, wenn die beiden Grundkörper gegen die Wirkung einer Druckfeder zusammengedrückt werden. Während des Zusammendrückens wird dann das in der Ampulle enthaltene Medium durch einen Auslasskanal ausgebracht. Es sind mehrere Ampullen am Umfang einer Trommel angeordnet, die zum zweiten Grundkörper gehört. Eine Weiterschaltung zwischen den einzelnen Ampullen findet durch einen Schaltmechanismus ähnlich einem Gesperre statt.

Die US 4,627,432 A beschreibt einen Spender, in den eine Blisterscheibe mit mehreren Speicherschalen am Umfang eingelegt wird. Durch eine Öffnung greift ein außen auf dem Gehäuse sitzendes Messer ein und durchlocht die jeweils unter dem Messer befindliche Speicherschale, so dass das darin enthaltene Medium in einen schalenartigen Raum fällt, aus dem es über ein Mundstück abgesaugt werden kann.

Der Erfindung liegt die Aufgabe zugrunde, einen Spender zu schaffen, bei welchem Nachteile bekannter Ausbildungen vermieden sind. Ferner sollen Medien auch in sehr niedriger volumetrischer Dosierung ausgebracht werden können. Der Spender soll sehr klein sein, z.B. mit einer menschlichen Hand vollständig abgedeckt umschlossen werden können. Er soll einfach in der Herstellung, der Montage, der Handhabung, der Aufbewahrung o. dgl. sein.

Diese Aufgabe wird durch den Anspruch 1 gelöst.

Der Spender kann zur Aufnahme eines Blisterpacks geeignet sein, der nur zwei Speicherkammern aufweist. Deren tiefgezogene Speicherschalen und eine Folienplatte bestehen aus einer durchsichtigen Kunststoffolie oder Metall, wie Aluminium. Die Schalenwandungen sind wesentlich dünner als die Platte, deren Dicke kleiner als ein halber Millimeter ist.

Die Schalenöffnungen sind mit einer demgegenüber dünneren Folie aus Kunststoff oder Metall, wie Aluminium, dicht verschlossen. Die ebene Schließfolie ist mit der ebenen Folienplatte thermoplastisch verschweißt. Die Schließfolie bildet in einem kordierten Raster Vorsprünge, welche unter Bildung entsprechender Vertiefungen in die Platte unlösbar eingeschmolzen sind.

Über die Rückseite der Platte stehen nur die Speicherschalen vor. Dadurch sind sie gut abzustützen. Der lichte Abstand zwischen den Schalen ist mindestens 1,5-fach größer als der Schalendurchmesser. Dieser ist größer als die Schalentiefe. Der Achsabstand zwischen den Schalen liegt unter 30 mm oder 25 mm.

Der Spender weist seitlich Fingervertiefungen bzw. eine Taille auf. Dadurch kann jeder Grundkörper zwischen Daumen und Zeigefinger sicher gegriffen werden; z.B. beim gegenseitigen Verdrehen der Grundkörper. Solche Vertiefungen bilden auch die voneinander abgekehrten Druckflächen zum axialen Verkürzen des Spenders.

Gegenüber dem Grundkörper ist die Speicherschale zweckmäßig anschließend an ihren Rand, am Außenumfang oder am Boden unmittelbar abgestützt. Dadurch ist ihre Lage genau festgelegt. Ferner ist sie so gegen Beschädigung abgestützt und abgeschirmt.

Zur genauen Drehausrichtung der beiden Grundkörper und des Speicherkörpers gegeneinander ist eine Schnappverbindung oder eine federnde Rast vorgesehen. Sie wirkt gleichermaßen in unterschiedlichen gegenseitigen Axialstellungen dieser drei Körper. Ihre Rastglieder können auch in einer oder mehreren gegenseitigen Axialstellungen dieser drei Körper einrasten. Jede Raststellung ist durch eine entsprechend hohe Dreh- oder Axialkraft überwindbar. Außerhalb der Raststellungen sind die Körper gegeneinander mit erhöhter Reibung schwergängig verdrehbar. In der Raststellung ist ein geringes, leichtgängigeres Drehspiel vorgesehen.

Die Ausgangsstellung für die Betätigung liegt zwischen der Drehstellung und der betätigten Endstellung. Zur Überführung in die Drehstellung ist in der Ausgangsstellung eine Rast zu überwinden. Sie kann durch die einander übergreifenden Ränder der Grundkörper gebildet sein. In der Drehstellung schlägt dann ein Abzieh-Anschlag an. Unter Aufbietung einer erhöhten Kraft kann auch dieser Anschlag, nämlich eine Schnappverbindung, überwunden werden, um die Grundkörper voneinander zu trennen. Von der Ausgangsstellung zur Endstellung ist kein Rastwiderstand zu überwinden, sondern nur der Öffnungswiderstand der Schließfolie und der Reibungswiderstand zwischen den Grundkörpern. In dieser Endstellung ist ein frei vorstehender Austragstutzen mindestens so lang wie der restliche Spender.

Der Spender ist so originalitätsgesichert, daß ohne Veränderung oder Zerstörung der Sicherung ein Speicherkörper eingesetzt werden kann, nicht jedoch dieser Speicherkörper dann geöffnet werden kann. Die Sicherung umgibt einen Rand des außen übergreifenden Grundkörpers vollständig und liegt staubdicht an diesem Rand an. Der Rand übergreift daher in der gesicherten Stellung den anderen Körper nicht. So können diese Körper nach Lösen der Sicherung ohne gegenseitige Axialbewegung sofort gegeneinander verdreht werden. Stehen dann die Grundkörper rechtwinklig zueinander, ist der Speicherkörper bzw. dessen Aufnahme frei sichtbar. Dies ist auch der Fall, wenn einer oder beide Grundkörper aus transparentem oder transluzentem Werkstoff bestehen.

Der Speicherkörper ist gegen axiales Herausziehen durch formschlüssig wirkende Mittel gesichert. Diese können mit einer erhöhten Kraft manuell überwunden werden. Die Sicherungsmittel für den Speicherkörper einerseits und zur Sicherung der Grundkörper aneinander andererseits wirken unabhängig voneinander und von der Originalitätssicherung.

Einschließlich jeder der genannten Sicherungen und ohne Speicherkörper besteht der Spender aus nur zwei jeweils einteiligen Teilen. Für deren gegenseitige Bewegung ist eine Rückstellfeder nicht erforderlich. Zur Einbeziehung von deren Merkmale und Wirkungen in die vorliegende Erfindung wird auf die deutsche Patentanmeldung 197 04 849.8 (= PCT/EP 9800311) Bezug genommen.

Unabhängig von der beschriebenen Ausbildung ist der Spender insbesondere zur Aufnahme und Lagerung biologischer Wirkstoffe über mehrere Wochen, Monate oder Jahre ausgebildet. Es können physiologische Wirkstoffe, wie hormonhaltige Wirkstoffe und/oder Wirkstoffe sein, die Spaltprodukte, wie Peptide, von Eiweiß enthalten. Solche biologischen Informationsüberträger, die Aminosäuren enthalten können und andere ähnliche Wirkstoffe können sehr feuchtigkeitsempfindlich sein. In dem Spender werden sie daher in einer druckdicht geschlossenen Kammer gelagert. Sie wird erst unmittelbar vor dem Austrag aus dem Spender geöffnet, z.B. durch Zerstörung eines Verschlusses.

Diese und weitere Merkmale der Erfindung gehen auch aus der Beschreibung und den Zeichnungen hervor, wobei die einzelnen Merkmale jeweils für sich allein oder zu mehreren in Form von Unterkombinationen bei einer Ausführungsform der Erfindung verwirklicht sein und vorteilhafte Ausführungen darstellen können. Ausführungsbeispiele der Erfindung sind in den Zeichnungen dargestellt und werden im folgenden näher erläutert. In den Zeichnungen zeigen:
- Fig. 1: einen erfindungsgemäßen Spender in Perspektive,

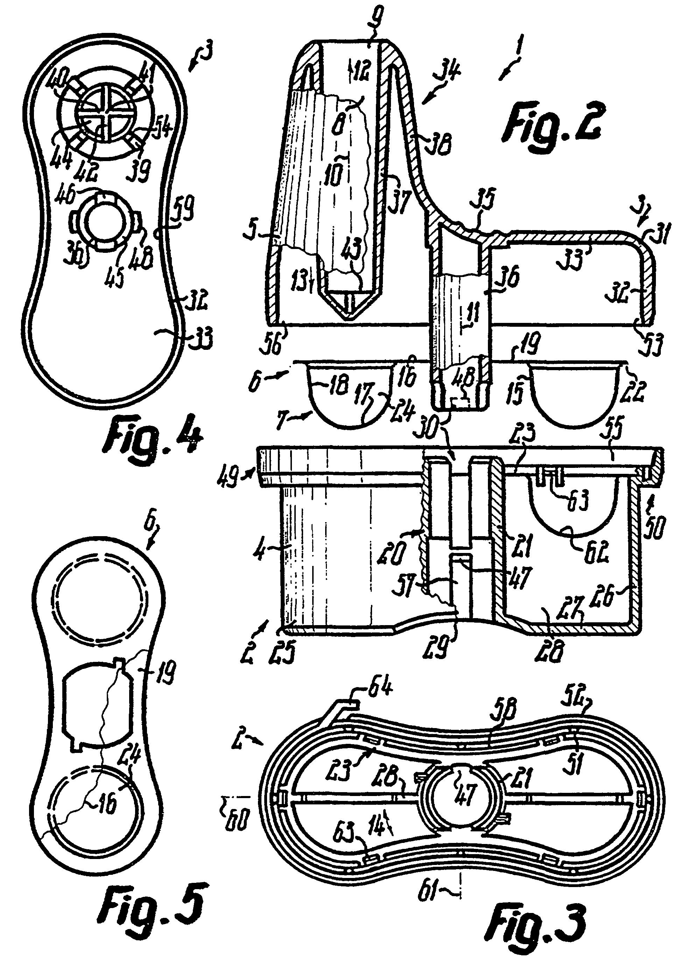

- Fig. 2: den Spender in explodierter und geschnittener Darstellung,
- Fig. 3: einen der Grundkörper in Draufsicht,
- Fig. 4: den zweiten Grundkörper in Unteransicht,
- Fig. 5: den Speicherkörper in Draufsicht,
- Fig. 6: eine weitere Ausführungsform in explodierter Perspektive und
- Fig. 7: eine dritte Ausführungsform in explodierter Perspektive.

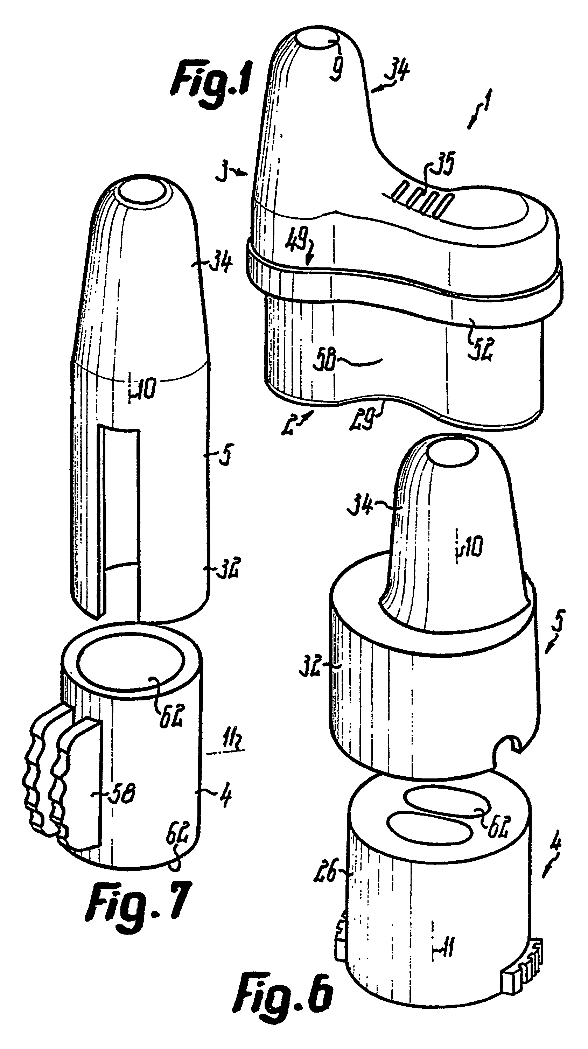

Der Spender 1 weist zwei Einheiten 2, 3 auf. Sie sind von einer Ausgangslage gemäß Fig. 1 unter Verkürzung des Spenders 1 und ohne gegenseitige Verdrehung in eine Endlage zu überführen. Ohne simultane Längenänderung sind die Einheiten 2, 3 gegeneinander in der Ausgangsstellung über mehr als 360° drehbar. Die erste Einheit 2 besteht nur aus einem becherförmigen Grundkörper 2 und einem vollständig innenliegenden Speicherkörper 6. Die zweite Einheit 3 besteht nur aus einem Grundkörper 5. Jeder Grundkörper 4 bzw. 5 ist einteilig.

Der Spender 1 dient zur Aufnahme von nur zwei gesonderten Mediendosen. Diese sind zeitlich aufeinanderfolgend auszutragen und in gesonderten Einzelspeichern 7 enthalten. Das Volumen des Speichers 7 ist mehr als vier-, sechs- oder achtfach größer als das enthaltene Medium.

Beim Austrag strömt das Medium durch den Speicher 7 unmittelbar in einen geradlinigen Auslaßkanal 8. Dessen kreisscheibenförmig freie Querschnitte nehmen in Strömungsrichtung spitzwinklig sowie stetig zu. Sein äußeres Ende bildet den Medienauslaß 9 und hat einen Durchmesser von mindestens 3 mm oder 5 mm. Die größte Speicherweite ist demgegenüber größer und kleiner als das 2,5- bzw. 1,5-fache.

Beim Austrag haben der Kanal 8, die Öffnung 9 und der eine Speicher 7 eine gemeinsame Achse 10. Sie liegt parallel zur Drehachse 11, zur Austragrichtung 12 und zur entgegengesetzten Betätigungsrichtung 13 der Einheit 3. Die zentrale Symmetrieachse der Einheiten 1 bis 6 liegt in zwei zueinander rechtwinkligen Symmetrieebenen. Zu diesen liegt die Drehrichtung 14 rechtwinklig.

Jeder Speicher 7 weist eine napfförmige Speicherschale 15 auf. Deren ebene Schalenöffnung ist mit einem Speicherverschluß 16, nämlich einer ebenen Membran druckdicht verschlossen. Der kugelkalottenförmige Schalenboden 17 geht stetig in einen Speichermantel 18 über. Dieser ist zur Speicheröffnung spitzwinklig konisch erweitert. Er geht unmittelbar in die ebene Speicherplatte 19 über, welche vollständig mit der Membran 16 abgedeckt und haftend verbunden ist.

Zwischen den einteiligen Speichern 7 ist die Platte 19 von einem teleskopartigen oder nur zweiteiligen Tragkörper 20 durchsetzt. Die Schalen 15 liegen im Radialabstand vom hohlen oder rohrförmigen Tragkörper 20. Dessen Außenumfang ist von einem frei vorstehenden Vorsprung 21 des Körpers 4 gebildet. Der Rand 22 der Platte 19 wird über seinen gesamten Umfang auf einer inneren Schulter 23 des Körpers 4 abgestützt. Zwischen den Speichern 7 wird die Platte 19 entlang einer zentralen Öffnung an einer Ringschulter des Domes 21 abgestützt. Dadurch sind die mikrodünnen Schalen 15 spielfrei lagegesichert, wenn ihre jeweilige Kammer 24 entleert wird. Bei gesondertem Körper 6 ist jede Wandung 15 und die Platte 16 bzw. 19 in sich biegeflexibel bzw. elastisch.

Der Körper 4 bildet eine in Stirnansicht längliche Kappe 25 mit einem äußersten Mantel 26 und einem Boden 27. Jede Schale 15 hat Abstand von beiden Wänden 26, 27. Gegenüber der offenen, ebenen Stirnfläche des Mantels 26 liegt die Stützfläche 23 um mindestens das Doppelte und höchstens das fünfoder vierfache der Dicke der Platte 19 versenkt.

In der gemeinsamen Axialebene 60 der Achsen 10, 11 sind Dorn 21, Mantel 26 und Boden 27 über unmittelbar an sie anschlieβende Rippen 28 versteift. Deren freie Kanten liegen in der Ebene der Fläche 23 und stützen die Platte 19 ebenfalls ab. Mit Abstand von und zwischen den Mänteln 21, 26 weist jedes Glied 28 eine vertiefte Aufnahme 62 auf. Sie ist eng an die Außenform der Schale 15 angepaßt und stützt daher die Bereiche 17, 18 unmittelbar streifenförmig ab.

Beim Einsetzen des Körpers 6 kommt zuerst der zentrale Durchbruch der Platte 19 in Eingriff mit dem Zentrierende des Dornes 21. Danach kommen gleichzeitig die Platte 19 und beide Schalen in Eingriff mit den Flächen 23, 62. Dabei wird eine Sicherung 63 wirksam, welche den Körper 6 formschlüssig daran hindert, bei umgedrehter Einheit 2 unter seinem Gewicht von den Flächen 23, 62 abzuheben. Die Sicherung weist am Innenumfang des über die Fläche 23 vorstehenden Randes des Mantels 26 Schnappvorsprünge 63 auf. Sie können radial federnd ausweichen, wenn der Rand 22 zwischen ihnen und der Fläche 23 spielfrei gehalten ist. Dabei steht der Körper 6 nicht über die Öffnung des Mantels 26 vor.

Der Körper 6 ist sowohl durch Anlage am Innenumfang des Mantels 26 als auch durch Eingriff des Dornes 21 jeweils für sich gegenüber den Körper 4 verdrehgesichert. Der Durchbruch in der Platte 19 weist hierfür eine von der Kreisform abweichende Form auf. Diese kann beispielsweise ein Kreis mit zwei einander gegenüberliegenden Abflachungen oder mit entgegengesetzt gerichteten Ausschnitten sein. An diese Form ist das Steckende des Dornes 21 komplementär angepaßt.

Die Außenseite der Wand 27 bildet gemeinsam mit dem nur an die Innenseite einteilig anschließenden Dorn 21 eine vertiefte Handhabe 29. Sie geht bis zu den Außenseiten beider Längsabschnitte des Mantels 26 durch, ist konkav gekrümmt und kann daher seitlich am letzten Gelenk des Zeigefingers zentriert abgestützt werden. Dabei stehen die beiden Speicherplätze frei über diesen Finger vor.

Die Verbindung 20 bildet gleichzeitig auch die zentrale Teleskopverbindung zwischen den Körpern 4, 5. Sie ist in jeder Relativlage der Körper 4, 5 nach außen vollständig abgedeckt. Sie dient als permanentes Drehlager 30, als Steckverbindung für die beiden Körper 4, 5 und zu deren gegenseitiger axialer Führung bei der Betätigung. Der Körper 5 bildet eine ebenfalls parallel zur Ebene 60 langgestreckte Kappe 31. Deren Mantel 32 steht von der Stirnwand 33 entgegengesetzt zum Mantel 26 in Richtung zur Wand 27 vor. Der Innenumfang des Mantels 33 ist an den Außenumfang des Mantels 26 eng angepaßt.

Die freie Endfläche des Domes 21 schlägt am Hubende an der Innenseite der Stirnwand 33 an. Dann sind zwischen den Innenseiten der Wandungen 32, 33 und den Außenseiten der Wandungen 26, 19 schlitzförmige Kanaldurchlässe 55 mit einem ringförmigen Einlaß 56 frei. Sie bilden Ansaugkanäle für Außenluft, die in einem Strömungsmantel um die Kammer 24 radial der Achse 10 zuströmt und dann in die Kammer 24 in Richtung 13 eingeleitet wird. Von dort strömt sie unter Mitnahme des Mediums zurück in Richtung 12 unmittelbar aus der Öffnung 9.

Die Ansaugung kann auch durch die Verbindung 20 bzw. 30 erfolgen und dann mit dem genannten Finger variabel gedrosselt werden, da der Durchlaß des Dornes 21 die Wand 27 nur im Bereich der Handhabe 29 durchsetzt.

Der Mantel 32 und die Stirnwand 33 gehen in einen in Richtung 12 frei vorstehenden Auslaßstutzen 34 über. Der steht über den ebenen Teil der Wand 33 um mehr als die Länge des Mantels 32 vor, z.B. um mindestens das 1,5-fache oder das Doppelte. Beiderseits der Ebene 60 und auf seiner von der Wand 33 abgekehrten Seite geht der Außenmantel 38 des Stutzens 34 über einen Bogenwinkel von mindestens 180° oder mehr ohne Zwischenschulter unmittelbar in den Mantel 32 über.

In die Wand 33 geht der Mantel 38 an der Außenseite konkav gekrümmt über und bildet hier deckungsgleich mit der Handhabe 29 eine vertiefte Handhabe 35. Sie weist Vorsprünge zur Erhöhung der Griffigkeit auf und dient zur Abstützung des Endes der Daumenkuppe, die gleichzeitig an der Wand 33 abgestützt sein und am Außenumfang des Stutzens 34 anschlagen kann. Von der Innenseite der Wand 35 steht ein Lagerdorn 36 vor. Er ist eng an den Innenumfang des Dornes 21 angepaßt und reicht in betätigter Endstellung bis zur Handhabe 29.

Die Handhabe 29 wird am Mittelfinger und die Handhabe 35 am Daumen derselben Hand abgestützt. So kann deren Zeigefinger am Außenumfang des Stutzens 34 abgestützt und der Spender 1 sehr sicher ausgerichtet werden. Der Dorn 36 steht über das Ende des Mantels 32 vor und ist von dem Anschlag für den Dorn 21 umgeben. Beim Schließen des Gehäuses 25, 31 kommen daher als erstes dessen Dorne 21, 36 in gegenseitigen Eingriff. Der Körper 6 kann zuerst auch auf den Dorn 36 aufgesteckt und beim Schließen des Gehäuses durch Schrägflächen von selbst über den Dorn 21 gestülpt werden. Die Dorne 21, 36 ragen entgegengesetzt frei aus.

Der Stutzen 34 weist auch einen inneren Mantel 37 auf. Er ragt frei entgegen Richtung 13 bis zur offenen Stirnfläche der Kappe 31 und hat in dieser Richtung bis zum inneren Ende stetig abnehmende Wandungsdicke. Sein äußeres Ende schließt in einer Ringzone einteilig an das äußere Ende des Mantels 38 an. Diese Ringzone begrenzt die Öffnung 9. Das Rohr 37 begrenzt den Kanal 8 und ist ab der Ringzone gegenüber dem Mantel 38 berührungsfrei. An seinem Außenumfang weist das Rohr 37 vier axiale Rippen 39 auf. Sie sind gleichmäßig über den Umfang verteilt und liegen in Axialebenen, welche unter 45° zur Ebene 60 geneigt sind. Die Rippen schließen an den Innenumfang der Mäntel 32, 38 nicht an.

Die Länge des Stutzens 34 über der Stirnwand 33 ist mindestens so groß wie der Abstand zwischen den Handhaben 29, 35 in der verkürzten Endstellung. Die ringförmige Endfläche des Stutzens 34 ist an ihrem Außenumfang mit größerem Radius abgerundet als an ihrem Innenumfang.

Beide Speicherkammern 24 sind mit der Folie 16 gemeinsam verschlossen. Sie deckt die Platte 19 vollständig ab. Zur Öffnung bildet das freie Ende des Rohres 37 ein rechtwinklig konisches Werkzeug 40. Dessen Spitze 41 geht in divergierende Stege 42 über. Sie schließen an die Endfläche des Rohres 37 mittig zwischen den Rippen 39 an. Zwischen benachbarten Trenngliedern 42 liegt jeweils eine Öffnung 44 eines Kanaleinlasses 43. In der verkürzten Endstellung ist die Schale 15 gegenüber den Teilen 37, 40 vollständig berührungsfrei. Die über die Wege 55, 56 angesaugte Luft tritt über den Ringkanal zwischen den Mänteln 18, 37 in die Kammer 24 ein, bildet dort eine Walzenströmung und strömt von dieser unmittelbar in den Kanal 8. Luft kann durch die Öffnung 9 auch in der Ruhelage angesaugt werden, also bevor das Werkzeug 40 den Verschluß 16 der zugehörigen Kammer 24 geöffnet hat. Nach dem Öffnen tauchen die Stege mit der Spitze 41 teilweise in das Medium ein. Dabei kann die Spitze 41 den Boden 17 gegen die Fläche 62 anlegen.

Das freie Ende des Dornes 36 bildet radial federnde Zungen 45, da sein Mantel in der Ebene 60 von zwei einander gegenüberliegenden Öffnungen 46 durchsetzt ist. Die Federenden 45 liegen beiderseits der Ebene 60 und weisen am Außenumfang jeweils einen vorstehenden Nocken 48 auf. Dem ist am Innenumfang des Dornes 21 und mit Abstand zwischen dessen Enden ein Gegenglied 47 zugeordnet. Es ist als Bodenvorsprung zwischen den Enden einer axialen Nut 57 vorgesehen. Sie reicht bis zu beiden Enden des Dornes 21.

Solche Nuten 57 mit Anschlägen 47 können einander gegenüberliegend am Innenumfang des Dornes 21 oder nur an einer Seite vorgesehen sein. In der Ausgangsstellung schlägt der jeweilige Nocken 48 am zugehörigen Anschlag 47 an. So ist eine Abzieh-Sicherung gebildet. Die Feder 45 ist dabei spannungsfrei und der Nocken 48 hat geringes Drehspiel. So ist auch in der Ausgangsstellung eine Drehsicherung gebildet.

Unter hinreichend erhöhter Abzugkraft wird der Nocken 48 an einer Schrägfläche des Anschlages 47 mit der Feder 45 radial nach innen gedrückt. So können die Körper 4, 5 vollständig getrennt werden. Der Körper 6 kann dann entnommen und ein gefüllter eingesetzt werden. Sobald der Dorn 36 beim Zusammenfügen den Dorn 21 erreicht hat, beginnt die Drehsicherung 47 wirksam zu werden. Bei weiterem Einschieben springt der Nocken 48 von selbst über den Nocken 47.

Werden in der Ausgangslage die Körper 4, 5 gegeneinander verdreht, so haben die freien Enden der Mäntel 26, 32 einen minimalen Spaltabstand voneinander, der gegenseitige Berührung erlaubt. Bei Drehung gleitet der Nocken 48 unter radialer Einwärtsfederung der Feder 45 über die Seitenflanke der Nut 57. Er gleitet dann mit erhöhter Reibung am Innenumfang des Dornes 21 bis er bzw. der andere Nocken 48 in die eine oder andere Nut 47 einspringt. Eine innere Ringschulter des Dornes 21 hält den Nocken 48 auch in jeder Drehstellung axial gesichert, jedoch mit erhöhter Kraft abziehbar.

Zur originalitätssicherung sind Mittel 49 vorgesehen, welche das Eingreifen der Sicherungen 47, 48, 57 wie auch das Trennen der Gehäuseteile 25, 31 erlauben, irgendeinen Teil des Arbeitshubes oder das gegenseitige Verdrehen der Körper 4, 5 jedoch sperren. Hierzu sind die einander zugekehrten Enden der Mäntel 26, 32 gemeinsam von einem flexiblen Band 52 eng umgeben, das einteilig mit dem inneren Mantel 26 ausgebildet ist und von beiden Mänteln 26, 32 geringen radialen Spaltabstand aufweist.

Dieser Spalt wird von Sollbruchgliedern 51 überbrückt. Sie nehmen in Richtung zur Sollbruchstelle am Außenumfang des Mantels 26 in der Breite und in der Dicke ab. Acht Glieder 51 sind gleichmäßig über den Umfang verteilt. Die Glieder 51 lösen sich bei Bruch glatt unmittelbar am geringfügig erweiterten Außenumfang des Mantels 26. An diesem Außenumfang kann dann der Innenumfang des Mantels 32 ungehindert auch an den Bruchstellen gleiten.

Das Band 52 ist in einem konvex gekrümmten Bereich an der Außenseite mit einem Axialschlitz versehen, welcher von einem abgewinkelten Vorsprung 64 überdeckt ist. In den Schlitz und unter den Haken 64 kann mit einem Fingernagel eingegriffen, das Glied 52 dann an dieser Stelle aufgebrochen und schließlich über den Umfang vollständig abgeschält werden. Auch durch kräftiges gegenseitiges Drehen der Körper 4, 5 läßt sich das Glied 52 absprengen.

Die axiale Betätigung kann in der Ausgangsstellung o. dgl. durch Mittel 50 blockiert sein. Sie werden durch eine erhöhte Druckkraft überwunden und erlauben dann eine wesentlich leichtergängige Verschiebung. Hierzu gleitet der offene Rand des Mantels 32 mit einer inneren Abschrägung 53 am Gegenrand des Mantels 26. Dabei wird der Mantel 32 federnd aufgeweitet. Im weiteren Verlauf durchsticht die Spitze 41 den Verschluß 16. Dann erst und nach Eindringen des Einlasses 43 schlitzen die Rippen 39 mit ihren Endkanten 54 den Verschluß 16 weiter auf.

Die Wände 26, 37, 32, 33 sind beiderseits der Achse 11 um die Achsen 10 der Speicher 7 über mehr als 180° kreisförmig gekrümmt und dazwischen eingeschnürt bzw. konkav tailliert. Dadurch sind an den Außenseiten der Mäntel 26, 32 konkave Vertiefungen 58 mit einem Krümmungsradius gebildet. Der ist größer als der Krümmungsradius um die Achsen 10. Die Vertiefungen 58 gehen zwischen sowie bis zu beiden Handhaben 29, 35 konstant durch. Sie erlauben ein sicheres Halten des Spenders 1 beim Drehen und Öffnen bzw. Schließen der Körper 4, 5.

An der Innenseite des Mantels 32 ist durch die Vertiefung 58 ein Vorsprung 59 gebildet. Er kann geringfügig näher bei der Ebene 60 als der zugehörige Bereich des Mantels 26 liegen. Der so gebildete Nocken 59 bewirkt dann den Druckpunkt der Steuerung 50, da er radial nach außen federn muß. Die Nocken 59 liegen in der zur Ebene 60 rechtwinkligen Mittel- oder Axialebene 61 der Achse 11. In dieser Ebene 61 liegen auch die Nocken 48, die Sicherungen 47, 57 und die Handhaben 29, 35. Auch die Platte 16, 19 weist die genannte taillierte Form auf.

Zum Gebrauch wird der Spender 1 geöffnet, ein Blisterpack 6 in den Körper 4 eingesetzt und dann der Körper 5 wieder axial auf den Körper 4 aufgesetzt, bis die Glieder 47, 48 einschnappen. Nun oder zuvor wird das Glied 52 abgerissen.

Unter Überwindung des Druckpunktes der Steuerung 50 werden die Körper 4, 5 zusammengedrückt. Dabei durchstößt die Spitze 41 den Verschluß 16 einer der beiden Kammern 24. Zuvor oder danach wird der Stutzen 34 in eine Nasenöffnung eingeführt. Durch Einatmen durch diese Nasenöffnung wird Luft über die Kanalwege 55, 56 angesaugt, nimmt das Pulver aus der Kammer 24 mit und gelangt von der Öffnung 56 bis zur Öffnung 9 ventilfrei in die Nasenöffnung.

Danach wird der Stutzen 34 aus der Nasenöffnung herausgezogen und die Körper 4, 5 werden wieder in ihre Ausgangsstellung manuell auseinandergezogen, indem sie z.B. an den Handhaben 58 gegriffen werden. Dann werden die Körper 4, 5 in Richtung 14 gegeneinander um 180° gedreht, so daß die zweite Kammer 24 achsgleich mit dem Stutzen 34 liegt. In derselben Weise wie im ersten Fall wird nun das Medium aus dieser Kammer 24 in die zweite Nasenöffnung gebracht. Auch hierbei ist die Steuerung 50 wirksam.

Gemäß Fig. 6 sind die beiden Mäntel 26, 32 der Körper 4, 5 zylindrisch und nur mit ihren Umfangsflächen unmittelbar aneinander geführt. Die Stutzenachse 10 liegt exzentrisch zur Achse 11 der Mäntel 26, 32. Die Speicheraufnahmen 62 liegen daher bis auf einen Spaltabstand unmittelbar benachbart zueinander, ohne daß zwischen ihnen ein Lager vorgesehen ist.

Gemäß Fig. 7 liegt die Wendeachse 11 rechtwinklig quer zur Achse 10 und kann diese schneiden. Der zylindrische Körper 4 weist an beiden voneinander abgekehrten Stirnseiten jeweils einen Speicherplatz 62 zur Aufnahme eines gesonderten Einzelspeichers auf. Nach dem Austrag aus dem ersten Einzelspeicher wird der Körper 4 vollständig vom Körper 5 gelöst, um seine Querachse 11 gewendet und wieder in den Körper 5 eingesetzt, um den zweiten Einzelspeicher zu entleeren. Der zylindrische Körper 5 bzw. dessen Mantel 32 steht hier radial nicht über den Stutzen 34 vor und liegt zu diesem achsgleich. Der Mantel 32 kann einen Längsschlitz für den herausragenden Eingriff der Handhabe 58 des Körpers 4 aufweisen.

Die dargestellten Größenverhältnisse sind besonders günstig. Alle Merkmale jeder Ausführungsform können bei jeder anderen Ausführungsform verwirklicht, nämlich hinzugefügt oder damit kombiniert sein. Die Eigenschaften und Wirkungen können genau oder nur im wesentlichen bzw. etwa wie beschrieben vorgesehen sein und je nach den Erfordernissen auch stärker davon abweichen.

Um zu vermeiden, dass geringe Medienmengen während des Durchbrechens oder vollständigen Öffnens des Verschlusses 16 mit dem Glied 40 ausgetragen werden, sind geeignete Mittel vorgesehen. Sie können eine Luftströmung oder Verdichtung in der Kammer 24 verhindern, welche sich durch Verkleinerung der Gehäusekammer ergibt, die gemeinsam von den Einheiten 2, 3 bzw. Körpern 4, 5 begrenzt ist. Dazu ist für diese Strömung ein temporärer Bypaß vorbei an der Kammer 24, den Öffnungen 9, 44 bzw. den Kanälen 8, 55, 56 vorgesehen. Der Bypaß kann Entweichöffnungen, wie Schlitze, aufweisen, welche mindestens eine der Wandungen 26, 27, 29, 32, 33, 35, 37, 38 direkt quer durchsetzen, die die Gehäusekammer begrenzen. Die Entweichöffnungen verbinden die Gehäusekammer direkt mit der Umgebungsatmosphäre und haben gemeinsam einen kleinsten Durchlaßquerschnitt, der größer ist bzw. einen Strömungswiderstand oder Kanallängen, die kleiner sind als der Durchlaßquerschnitt bzw. der Strömungswiderstand oder die Kanallängen des Kanales 55, 56. Die Mittel können auch einen Zusatzverschluß zum temporären Verschließen der Öffnung der Kammer 24, des Kanales 8 bzw. des Auslasses 9 umfassen. Es können auch Mittel vorgesehen sein, um automatisch die mindestens eine Entweichöffnung zu schließen bzw. um automatisch nach anfänglichem Schließen die Zusatzverschlüsse darauffolgend zu öffnen, nämlich am Ende des Öffnungs-Teilhubes sowie durch die manuelle Betätigungskraft, welche die Hubbewegung bewirkt. Erst nach dem Öffnungs-Teilhub ist dann der Stutzen für den Austrag in die Nasenöffnung einzuführen.

## Patentansprüche

1. Spender für Medien,
1.1 mit zwei Grundkörpern (4, 5), einem Auslaßkanal (8) und einem Medienauslaß (9),
1.2 sowie mit wenigstens zwei jeweils in einer Kammer (4) eine Austragdosis des Mediums versiegelt aufnehmenden Speicherschale (15),
1.3 in die beim Medienaustrag ein an einem der Grundkörper (5) vorgesehener Kanaleinlaß (43) des Auslaßkanales (8) zur Öffnung eines Speicherverschlusses (16) eingreift,
1.4 wobei die Grundkörper (4, 5) axial von einer Ausgangsstellung in eine Endstellung und
1.5 drehend zwischen wenigstens zwei Drehstellungen gegeneinander bewegbar sind,
**dadurch gekennzeichnet, dass**
1.6 die die Kammern (24) aufweisenden Speicherschalen (15) Teil eines Speicherkörpers (6) sind,
1.7 dass in der Endstellung Kanalwege (55) für eine in die Kammer (24) einer ersten Speicherschale (15) des Speicherkörpers (6) gerichtete Einlass-Strömung gebildet sind,
1.8 und dieser Endstellung der Auslasskanal (8) für den Medienaustrag durch Ansaugen des Mediums an die Kammer (24) angeschlossen ist und
1.9 dass die Grundkörper (4, 5) manuell in ihre Ausgangsstellung auseinander ziehbar sind,
1.10 und in dieser Ausgangsstellung manuell in eine andere der Drehstellungen drehbar sind, in der eine Kammer (24) einer zweiten Speicherschale (15) des Speicherkörpers (6) für den Medienaustrag angeordnet ist,
1.11 wobei jeweils eine Rast (48, 57) die Grundkörper (4, 5) in dieser Drehstellung sichert.

2. Spender nach Anspruch 1, **dadurch gekennzeichnet, daß** mindestens ein Grundkörper (4, 5) an wenigstens einer Außenseite eine Finger-Vertiefung (58) für den Eingriff einer Fingerkuppe des Benutzers aufweist, die sich zwischen den Achsen (10) zweier Speicherschalen (15) erstreckt.

3. Spender nach Anspruch 1 oder 2, **dadurch gekennzeichnet, daß** mindestens ein Grundkörper (4, 5) in Axialansicht zwischen zwei Enden länglich und etwa ab der Mitte seiner Länge zu wenigstens einem der Enden spitzwinklig verbreitert ist und daß mindestens eines der Enden kreisbogenförmig ist.

4. Spender nach einem der vorhergehenden Ansprüche, **dadurch gekennzeichnet, daß** mindestens eine Vertiefung (29, 35, 58) an der Außenseite des Spenders (1) konkav gekrümmt ist, und daß vorzugsweise zwei voneinander abgekehrte Vertiefungen die Betätigungs-Handhaben (29, 35) zur axialen Betätigung und zwei voneinander abgekehrte Vertiefungen Handhaben (58) zum Öffnen bzw. in sich Verdrehen des Spenders (1) bilden.

5. Spender nach einem der vorhergehenden Ansprüche, **dadurch gekennzeichnet, daß** insbesondere die Rast einen axialen Anschlag (47) der Grundkörper (3, 4) bildet, und daß die Rast beim gegenseitigen Verdrehen der Grundkörper (4, 5) eine Sicherung gegen gegenseitiges Abziehen der Grundkörper (4, 5) bildet.

6. Spender nach einem der vorhergehenden Ansprüche, **dadurch gekennzeichnet, daß** der Grundkörper (4) zur Abstützung des Speicherkörpers (6) eine vertiefte Stützschulter (23) aufweist, daß der Grundkörper (4) zur Lagesicherung des Speicherkörpers (6) mindestens ein Schnappglied (63) aufweist.

7. Spender nach einem der vorhergehenden Ansprüche, **dadurch gekennzeichnet, daß** der Grundkörper (4) für den Boden (17) der Speicherschale (15) eine die Schalenachse (10) überbrückende Abstützung (62) aufweist, die durch eine vertiefte Kantenfläche einer Rippe (28) gebildet ist.

8. Spender nach einem der vorhergehenden Ansprüche, **dadurch gekennzeichnet, daß** eine Stützschulter (23) für den Speicherkörper (6) eine äußere, in Axialansicht in entgegengesetzten Richtungen gekrümmte Ringschulter zur Abstützung des Randbereiches des Speicherkörpers (6) aufweist, die eine innere Ringschulter zur Abstützung des Speicherkörpers (6) um eine zentrale Öffnung aufweist.

9. Spender nach einem der vorhergehenden Ansprüche, **dadurch gekennzeichnet, daß** an dem Grundkörper (4) ein gegen Betätigung formschlüssig sicherndes, ringförmiges Abreißband (52) vorgesehen ist, das mit dem Grundkörper (4) über radiale Sollbruchglieder (51) einteilig verbunden ist, die zum Grundkörper (4) in der Dicke und/oder Breite abnehmen.

10. Spender nach einem der vorhergehenden Ansprüche, **dadurch gekennzeichnet, daß** ein erster Grundkörper (4) zur verdrehgesichert feststehenden Aufnahme des Speicherkörpers (6) sowie zur drehbaren Steckverbindung mit einem zweiten Grundkörper (5) ausgebildet ist, welcher den Medienauslaß (9) aufweist, daß der zweite Grundkörper (5) mit einem radial federnden Lagerdorn (36) in den ersten Grundkörper (4) eingreift sowie den Speicherkörper (6) durchsetzt, und daß der Spender (1) zur auswechselbaren Aufnahme des Speicherkörpers (6) aus nur zwei Grundkörpern (4, 5) besteht, von denen jeder einteilig ausgebildet ist.

11. Spender nach einem der vorhergehenden Ansprüche, **dadurch gekennzeichnet, daß** der Spender zur Speicherung und zum Austrag biologischer bzw. ähnlicher Wirkstoffe, insbesondere zum Austrag physiologischer Wirkstoffe, wie hormonhaltiger Wirkstoffe ausgebildet ist, vorzugsweise zum Austrag von Wirkstoffen, wie Peptiden, die Spaltprodukte von Eiweiß enthalten.

## Claims

1. A dispenser for media comprising
1.1 two base bodies (4, 5), an outlet passage (8), and a medium orifice (9)
1.2 as well as at least two blister cups (15) each sealingly accommodating a discharge dose of said medium in a blister (4),
1.3 a passage inlet (43) of said outlet passage (8) engaging one of said base bodies (5) on discharge of said medium to open a blister seal (16)
1.4 said base bodies (4, 5) being axially moveable from a starting position into an end position and
1.5 rotatively moveable between at least two rotary positions opposingly,
**characterized in that**
1.6 said blister cups (15) comprising said blisters (24) are part of a blister pack (6),
1.7 in said end position passageways (55) are formed for an inlet flow directed into said blister (24) of a first blister cup (15) of said blister pack (6),
1.8 and that in said end position said outlet passage (8) for discharge of the medium by suction of the medium is connected to said blister (24) and
1.9 that said base bodies (4, 5) can be manually pulled apart into their starting position,
1.10 and in this starting position are manually rotatable into another of said rotary positions in which a blister (24) of a second blister cup (15) of said blister pack (6) is arranged for medium discharge,
1.11 one latch (48, 57) each locking said base bodies (4, 5) in said rotary position.

2. The dispenser as set forth in claim 1, **characterized in that** at least one base body (4, 5) comprises on at least one outer side a finger hold dishing (58) for engaging a finger tip of the user, said finger hold dishing (58) extending between the centerlines (10) of two blister cups (15).

3. The dispenser as set forth in claim 1 or 2, **characterized in that** at least one base body (4, 5) as viewed axially is flared longitudinally between two ends at an acute angle to at least one of said ends as of roughly the middle of its length, and that at least one of said ends has the shape of a circular arc.

4. The dispenser as set forth in any of the preceding claims, **characterized in that** at least one actuating finger hold (29, 35, 58) forms a concave curve at the outer side of said dispenser (1), and that preferably two dishings facing away from each other form said actuating finger holds (29, 35) for axial actuation and two dishings facing away from each other form finger holds (58) for opening or twisting said dispenser (1).

5. The dispenser as set forth in any of the preceding claims, **characterized in that** more particularly said latch forms an axial stop (47) on said base body (4, 5) and that said latch forms a captive safeguard preventing mutual removal of said base bodies (4, 5) in mutual twisting said base bodies (4, 5).

6. The dispenser as set forth in any of the preceding claims, **characterized in that** for supporting said blister pack (6) said base body (4) comprises a recessed supporting shoulder (23), for locking in place said blister pack (6) said base body (4) comprising at least one action member (63).

7. The dispenser as set forth in any of the preceding claims, **characterized in that** for receiving the bottom (17) of said blister cup (15) said base body (4) comprises a support (62) bridging said centerlines, said support (62) being formed by a recessed edge surface area of a rib (28).

8. The dispenser as set forth in any of the preceding claims, **characterized in that** a supporting shoulder (23) for said blister pack (6) comprises an outer ring shoulder curved in opposing directions, as viewed axially, for supporting the edge portion of said blister pack (6), said supporting shoulder (23) comprising an inner ring shoulder for supporting said blister pack (6) about a central opening.

9. The dispenser as set forth in any of the preceding claims, **characterized in that** a ring-shaped peel tag (52) positively preventing actuation is provided on said base body (4), said peel tag (52) being integrally connected to said base body (4) by radial design-break members (51) having in the direction of said base body (4) a reduced thickness and/or width.

10. The dispenser as set forth in any of the preceding claims, **characterized in that** a first base body (4) is configured for receiving said blister pack (6) in a fixed anti-twist location as well as for twistingly connecting a second base body (5) comprising said medium orifice (9), said second base body (5) engaging said first base body (4) by a radially flexing arbor (36) passing through said blister pack (6) and said dispenser consisting of only two base bodies (4, 5) for interchangeably receiving said blister pack (6), each base body (4, 5) being configured integrally.

11. The dispenser as set forth in any of the preceding claims, **characterized in that** said dispenser is configured for storing and discharging biological or similar active substances, particularly physiological active substances such as hormonal active substances and preferably for discharging active substances such as peptides containing breakdown products of protein.

## Revendications

1. Distributeur de substances
1.1 avec deux corps de base (4, 5), un conduit de sortie (8) et un moyen d'échappement de substance (9),
1.2 ainsi qu'avec au moins deux cuvettes de réservoir (15), logeant de manière scellée respectivement dans une chambre (4) une dose de substance à décharger,
1.3 dans lesquelles s'engage pendant la décharge de la substance un accès de conduit (43) du conduit de sortie (8) prévu sur un des corps de base (5) pour l'ouverture d'une fermeture de réservoir (16),
1.4 les corps de base (4, 5) étant movibles l'un par rapport à l'autre en direction axiale d'une position initiale dans une position terminale et
1.5 en direction de rotation entre au moins deux positions angulaires,
**caractérisé en ce que**
1.6 les cuvettes de réservoir (15) qui présentent les chambres (24) font partie d'un corps de réservoir (6),
1.7 **en ce que** dans la position terminale des voies de conduit (55) sont formées pour un écoulement d'accès orienté à l'intérieur de la chambre (24) d'une première cuvette de réservoir (15) du corps de réservoir (6),
1.8 et que dans cette position terminale le conduit de sortie (8) est raccordé à la chambre (24) pour la décharge de la substance par aspiration de la substance et
1.9 **en ce que** les corps de base (4, 5) peuvent être réciproquement étendus manuellement pour atteindre leur position initiale,
1.10 et que dans cette position initiale il sont orientables manuellement dans une autre position angulaire, dans laquelle une chambre (24) d'une deuxième cuvette de réservoir (15) du corps de réservoir (6) est disposée pour la décharge de la substance,
1.11 un cran d'arrêt (48, 57) assurant respectivement les corps de base (4, 5) dans cette position angulaire.

2. Distributeur d'après la revendication 1, **caractérisé en ce qu'**au moins un corps de base (4, 5) présente au moins à une des faces extérieures un creux à doigts (58) pour l'engagement d'un bout du doigt de l'utilisateur, le creux s'étendant entre les axes (10) de deux cuvettes de réservoir (15).

3. Distributeur d'après la revendication 1 ou 2, **caractérisé en ce qu'**au moins un corps de base (4, 5) est élargi en vue axiale de manière oblongue entre deux extrémités et à peu près à partir de la moitié de sa longueur de manière acutangulaire au moins en direction d'une des extrémités et **en ce qu'**au moins une des extrémités présente la forme d'un arc de cercle.

4. Distributeur d'après une des revendications précédentes, **caractérisé en ce qu'**au moins un creux (29, 35, 58) est courbée de manière concave à la face extérieure du distributeur (1), et **en ce que** de préférence deux creux opposés forment les manettes d'actionnement (29, 35) pour l'actionnement axial et que deux creux opposés forment des manettes (58) pour l'ouverture ou encore pour le déplacement angulaire du distributeur (1) en soi-même.

5. Distributeur d'après une des revendications précédentes, **caractérisé en ce que** notamment le cran forme un arrêt (47) axial des corps de base (3, 4), et **en ce que** le cran d'arrêt forme un assurage contre un détachement réciproque des corps de base (4, 5) pendant la rotation réciproque des corps de base (4, 5).

6. Distributeur d'après une des revendications précédentes, **caractérisé en ce que** le corps de base (4) présente un épaulement de support (23) creusé pour le soutien du corps de réservoir (6), **en ce que** le corps de base (4) présente au moins un élément à déclic (63) pour l'assurage de la position du corps de réservoir (6).

7. Distributeur d'après une des revendications précédentes, **caractérisé en ce que** pour le fond (17) de la cuvette de réservoir (15) le corps de base (4) présente un appui (62) qui jette un pont par-dessus l'axe de cuvette (10) et qui est formé par une surface d'arête creuse d'une nervure (28).

8. Distributeur d'après une des revendications précédentes, **caractérisé en ce qu'**un épaulement de support (23) d'un corps de récipient (6) présente, pour le soutien du domaine marginal du corps de récipient (6), un épaulement annulaire extérieur, courbé dans des directions opposées en vue axiale, qui présente un épaulement annulaire intérieur pour le soutien du corps de réservoir (6) autour d'une ouverture centrale.

9. Distributeur d'après une des revendications précédentes, **caractérisé en ce que** sur le corps de base (4) on prévoit une bande de rupture (52) annulaire assurant à engagement positif contre tout actionnement, qui est raccordée d'une seule pièce au corps de base (4) au moyen d'éléments radiaux destinés à la rupture (51), dont l'épaisseur et/ou la largeur se réduit en direction du corps de base (4).

10. Distributeur d'après une des revendications précédentes, **caractérisé en ce qu'**un premier corps de base (4) est réalisé de manière appropriée pour le logement immobile assuré contre rotation, du corps de réservoir (6) ainsi que pour le raccordement rotatif par enfichage avec un deuxième corps de base (5), lequel présente le moyen d'échappement de substance (9), **en ce que** le deuxième corps de base (5) s'engage dans le premier corps de base (4) au moyen d'une broche de logement (36) radialement élastique, ainsi qu'il traverse le corps de support (6), et **en ce que** pour le logement interchangeable du corps de réservoir (6) le distributeur (1) n'est composé que de deux corps de base (4, 5), dont chacun est réalisé d'une seule pièce.

11. Distributeur d'après une des revendications précédentes, **caractérisé en ce que** le distributeur est réalisé de manière à convenir à l'emmagasinage et à la décharge d'agents biologiques ou encore de substances similaires, notamment pour la décharge d'agents physiologiques, tels que des médicaments hormonaux, de préférence pour la décharge d'agents, tels que des peptides, qui contiennent des produits de protéolyse.
